# EUROPEAN PATENT SPECIFICATION

(11) **EP 4 646 237 B1**
(45) Date of publication and mention of the grant of the patent: **04.11.2026**
(21) Application number: 24705265.7
(22) Date of filing: 04.01.2024
(51) Int. Cl.: A61L 2/18, A61B 90/70, A61L 2/28, G01N 31/22, A61L 2/24

(54) **PROCESS INDICATOR ISOLATION CHAMBER FOR MEDICAL DEVICE PROCESSOR**
CHAMBRE D'ISOLATION D'INDICATEUR DE PROCESSUS POUR PROCESSEUR DE DISPOSITIF MÉDICAL
CHAMBRE D'ISOLATION D'INDICATEUR DE PROCESSUS POUR PROCESSEUR DE DISPOSITIF MÉDICAL

(30) Priority: 08.01.2023 US 202363478983 P
(43) Date of publication of application: 12.11.2025
(73) Proprietor: American Sterilizer Company, Mentor, OH 44060 (US)
(72) Inventor: LANGE, Ethan K., Painesville, Ohio 44077 (US)
(74) Representative: FRKelly
(86) International application number: PCT/US2024/010240
(87) International publication number: WO 2024/148124

(56) References cited:
- WO-A1-2022/263950
- US-A- 4 839 291
- US-A- 5 736 355
- US-A- 5 866 356
- US-A1- 2014 262 829

## Description

### Field of Invention

This application relates generally to a system and method for determining the efficacy of a disinfection and/or sterilization process, and more particularly to a process indicator isolation chamber for a medical device processor and method of using the same.

### Background

Medical device processors, also referred to as medical instrument processors, are widely used in various health care settings and are typically associated with high-level disinfection or sterilization of medical instruments. An example of such a processor is an automated endoscope reprocessor (AER) used for reprocessing endoscopes, such as duodenoscopes, and endoscope accessories. AERs are designed to kill microorganisms in or on reusable endoscopes by exposing their outside surfaces and interior channels to liquid chemical sterilant or high-level disinfectant solutions.

One parameter that determines the efficacy of the disinfection and/or sterilization process for AERs is the concentration of the disinfectant or sterilant used during the process. In some implementations, the concentration of the disinfectant or sterilant in solution can be verified using chemical indicator strips, which are designed to change color based on the disinfectant/sterilant concentration in solution. A chemical indicator strip can be checked against a color scale to determine if the medical instrument was processed with a solution having the disinfectant or sterilant above the minimum required concentration for disinfection/sterilization efficacy.

In some examples, an end user of the AER manually mixes the disinfectant/sterilization solution and dips the chemical indicator strip in the solution for a set period of time. In other examples, the AER dispenses a volume of the disinfectant/sterilization solution and is used by an end user by dipping the chemical indicator strip therein for a set period of time, in which case the user has to return to the machine during the cycle to dip the chemical indicator strip. However, these approaches can be burdensome on the end user and/or also do not necessarily provide the end user with an accurate indication of the concentration of the disinfectant/sterilization solution as used in the process. In still other examples, the chemical indicator strip can be included in the basin of the AER (e.g., in the processing tray of the basin) during the disinfection/sterilization cycle. This approach allows for detection of the concentration of the solution actually used during the cycle. However, AERs have been developed that integrate a cleaning cycle with the disinfection/sterilization cycle as an additional part of the process. As a result, the AER utilizes multiple different chemistries during the respective processing cycles. This presents a challenge for chemical indicator test strips that are used in the tray/basin during the processing cycle, because the chemical indicator will be exposed to the cleaning chemistry before the disinfectant/sterilant is deployed. The chemical indicator can be damaged or otherwise compromised such that the color change is not accurate or reliable. Similar issues are also presented for other biological indicators (e.g., spore test strips) that are included in the basin of the AER during the cycle, as they can be compromised by the cleaning chemistry and any associated microbial wash-off. WO 2022/263950 and US 5866356 disclose known medical device processors.

Accordingly, there remains a need for further contributions in this area of technology.

### Summary of Invention

The application relates to a process indicator isolation chamber for a medical device processor. The outlet of the process indicator isolation chamber is in fluid communication with the open basin of the medical device processor and is configured to allow process indicators to be used during a processing cycle performed by the medical device processor. The process indicator isolation chamber can isolate one or more process indicators in the isolation chamber from liquid used during a cleaning process such that the one or more process indicators are not exposed to cleaning chemistries used in connection with the cleaning cycle. The inlet of the process indicator isolation chamber can be selectively opened when the disinfectant/sterilant is deployed during the disinfecting/sterilizing cycle to allow the liquid including the disinfectant/sterilant to flow through the isolation chamber, come into contact with the one or more process indicators, and exit the isolation chamber via the outlet into the basin.

The process indicator isolation chamber allows for one or more process indicators to be used in fluid communication with the basin during a process including both cleaning and disinfecting/sterilizing steps. This reduces the manual burden on the user by negating the need to manually prepare a bulk chemistry solution and test said solution and/or by negating the need to manually test a volume of disinfectant/sterilization solution during the cycle, and also reduces the possibility of human error. The process indicator isolation chamber allows for the selective exposure of the one or more process indicators during the process. This negates the need for the user to separately expose the one or more process indicators to a dispensed volume of disinfection/sterilization solution during the process.

In accordance with one aspect of the present disclosure, a process indicator isolation chamber includes: a housing having an interior volume, the housing including: an inlet at a first end of the housing; an opening at a second end of the housing opposite the first end; a flange extending radially outward from an outer surface of the housing, proximate the second end; and a removable module including: a frame at least partially disposed in the interior volume of the housing, the removable module including a first end disposed in the interior volume of the housing; a shielding cap attached to the frame at a second end of the removable module opposite the first end of the removable module, the shielding cap including a top portion that covers the opening of the housing and a side portion that covers a portion of the outer surface of the housing between the flange and the second end of the housing; and a process indicator mount attached to the frame and disposed in the interior volume of the housing, the process indicator mount configured to hold at least one test indicator strip in a fluid path between the inlet and the opening; wherein a tortuous outlet passage is formed between the shielding cap and the outer surface of the housing.

In some embodiments, the process indicator isolation chamber includes: a first magnet attached to the first end of the frame; and a second magnet attached to the housing proximate the second end of the housing, wherein the first magnet and the second magnet interact to retain the module in the housing via magnetic force.

In some embodiments, the process indicator isolation chamber includes a switch configured to detect the insertion state of the removable module in the housing. In some embodiments, the switch is a reed switch.

In some embodiments, the process indicator mount includes a clip mounting post configured to mount a clip holding a process indicator.

In some embodiments, the process indicator mount includes a clip configured to hold a process indicator.

In some embodiments, the process indicator mount includes a spring clip.

In some embodiments, the process indicator mount is a first process indicator mount, and the removable module includes a second process indicator mount configured to hold an additional test indicator strip.

In some embodiments, the frame includes: a main body having opposed major surfaces; and side rails extending perpendicular to the main body, the side rails and the main body forming a channel, wherein the process indicator mount is arranged to provide the process indicator at least partially in the channel.

In some embodiments, the process indicator isolation chamber includes one or more cut out regions extending through the main body, the one or more cut out regions configured to allow fluid flow through the main body.

In some embodiments, the shielding cap includes offset gussets extending from the inner surface of the top portion to maintain a gap between the second end of the housing and the shielding cap.

In accordance with another aspect of the present disclosure, a medical device processor includes: a basin assembly including a basin tub; and the process indicator isolation chamber as described above in accordance with the one aspect of the present disclosure disposed in a through hole of the basin tub, the inlet of the process indicator isolation chamber housing positioned outside the basin, the opening at the second end of the housing positioned inside the basin tub, wherein the process indicator isolation chamber is fluidly connected with the basin such that fluid can flow through the isolation chamber into the basin tub.

In some embodiments, wherein the basin assembly further includes: a lid configured to selectively cover the basin tub; one or more nozzles in fluid communication with the internal volume of the basin tub; one or more connectors in fluid communication with the internal volume of the basin tub and configured to connect to a medical device processed in the medical device processor; one or more spray bars in fluid communication with the interior volume of the basin tub; and a drain in fluid communication with the interior volume of the basin tub, wherein the tortuous outlet passage of the process indicator isolation chamber is in fluid communication with the interior volume of the basin tub.

In some embodiments, the medical device processor includes: a manifold; and a supply line connecting the manifold to the one or more connectors; a pump and/or valve to control flow of fluid to the one or more connectors; a supply line connecting the manifold to the one or more spray bars; a pump and/or valve to control flow of fluid to the one or more spray bars; a supply line connecting the manifold to the process indicator isolation chamber; a pump and/or valve to control flow of fluid to the process indicator isolation chamber; a return line connecting the drain to the manifold; and a pump and/or valve to control flow of fluid from the drain of the basin assembly to the manifold.

In some embodiments, the medical device processor includes: a controller configured to control the respective valves and/or pumps of the supply lines and the pump and/or valve of the return line.

In some embodiments, the controller is configured to execute a processing cycle program stored on a memory, the processing cycle program including: a cleaning cycle in which a solution including detergent is cycled through the basin tub and manifold for a predetermined amount of time by introducing the solution including detergent from the manifold to the basin tub via the one or more spray bars, and one or more connectors, and by draining the solution from the basin back to the manifold, the pump and/or valve associated with the process indicator isolation chamber being controlled by the controller such that the solution including the detergent is not introduced into the process indicator isolation chamber; and a decontamination cycle in which a solution including a decontaminant is cycled through the basin tub and manifold for a predetermined amount of time by introducing the solution including the decontaminant from the manifold to the basin tub via the one or more spray bars, and one or more connectors, and by draining the solution including the decontaminant from the basin back to the manifold, the pump and/or valve associated with the process indicator isolation chamber being controlled by the controller such that the solution including the decontaminant is introduced into the process indicator isolation chamber.

In some embodiments, the decontaminant is a high-level disinfectant.

In some embodiments, the decontaminant is a sterilant.

In some embodiments, the decontaminant is peracetic acid or glutaraldehyde.

In accordance with another aspect of the present application, a method of determining the efficacy of a disinfection process includes the steps of: performing a cleaning cycle in a medical device processor, the medical device processor including: a basin assembly including a basin tub; a lid configured to selectively cover the basin tub; one or more nozzles in fluid communication with the internal volume of the basin tub; one or more connectors in fluid communication with the internal volume of the basin tub and configured to connect to a medical device processed in the medical device processor; one or more spray bars in fluid communication with the interior volume of the basin tub; and the process indicator isolation chamber as described above in accordance with the one aspect of the present disclosure disposed in a through hole of the basin tub, the inlet of the process indicator isolation chamber housing positioned outside the basin, the opening at the second end of the housing positioned inside the basin tub, wherein the process indicator isolation chamber is fluidly connected with the basin such that fluid from the basin can be selectively flowed through the isolation chamber, wherein the cleaning cycle includes introducing solution including detergent into to the basin tub via the one or more spray bars, one or more nozzles, and one or more connectors, the solution including the detergent not being introduced into the process indicator isolation chamber during the cleaning cycle; and performing a decontamination cycle including introducing solution including decontaminant into to the basin tub via the one or more spray bars, one or more nozzles, and one or more connectors, the solution including the decontaminant being introduced into the process indicator isolation chamber during the decontamination cycle.

The following description and the annexed drawings set forth certain illustrative embodiments of the invention. Other objects, advantages, and novel features according to aspects of the invention will become apparent from the following detailed description when considered in conjunction with the drawings.

### Brief Description of the Drawings

The annexed drawings, which are not necessarily to scale, show various aspects of the invention.
FIG. 1A is a schematic view of parts of an exemplary medical device processor.
FIG. 1B is a schematic view of parts of an exemplary medical device processor.
FIGS. 2 and 3 are schematic views of a portion of the basin of the medical device processor including an isolation chamber.
FIG. 4 is a schematic side view of the isolation chamber with the module secured in the chamber.
FIG. 5 is a schematic perspective view of the isolation chamber with the module removed from the chamber.
FIG. 6 is a schematic cross-sectional view of the isolation chamber with the module secured in the chamber.
FIG. 7 is a schematic cross-sectional view of a portion of the isolation chamber showing a flow control orifice of the chamber.
FIG. 8 is a schematic perspective view of parts of the module.
FIG. 9 is a computational fluid dynamics (CFD) flow velocity magnitude contour of the flow path through the isolation chamber with the module inserted in the housing.
FIGS. 10 and 11 are schematic cross-sectional views of portions of the isolation chamber showing the flow path formed by the shield cap of the module and the housing.
FIG. 12 is a CFD flow velocity magnitude contour of the tortuous outlet passage formed by the shield cap of the module and the housing.
FIG. 13 is a schematic view of an exemplary control system.
FIG. 14 is a flowchart showing an exemplary process including both cleaning and disinfecting/sterilizing cycles.

### Detailed Description

While the present invention can take many different forms, within the scope of the claims, for the purpose of promoting an understanding of the principles of the invention, reference will now be made to the embodiments illustrated in the drawings and specific language will be used to describe the same.

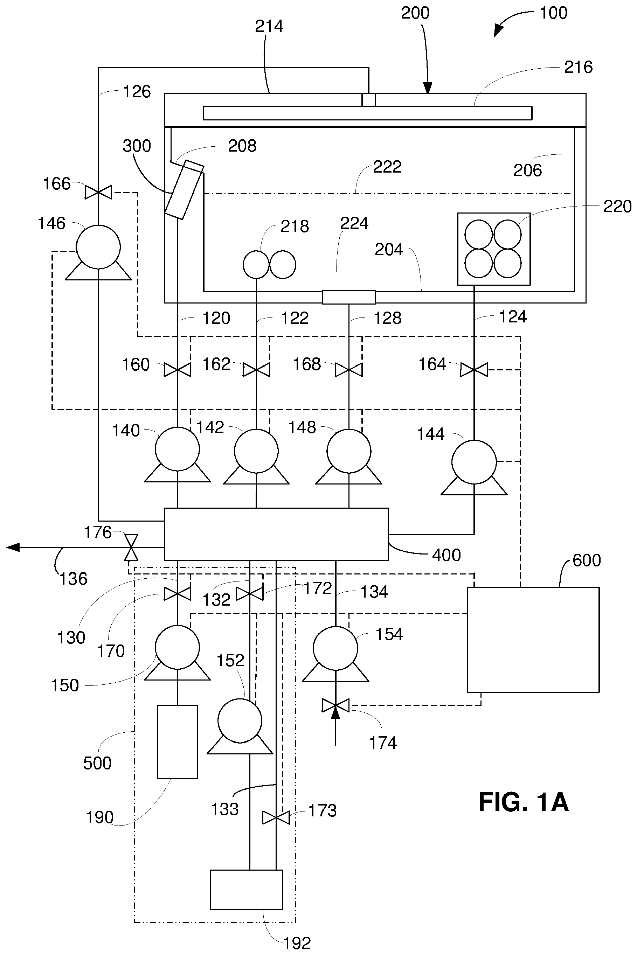

Turning now to the drawings, and initially to FIG. 1A, an exemplary medical device processor is shown at 100. The medical device processor 100 is configured to clean and decontaminate one or more medical devices inserted into the medical device processor 100. The medical device processor 100 may be any type of system for cleaning and decontaminating medical devices or instruments, for example, by a cleaning process and a high-level disinfection process and/or sterilization process. A high-level disinfection process and/or sterilization process will also be generally referred to herein as a decontamination process. In an exemplary embodiment, the medical device processor 100 is an automated endoscope reprocessor (AER) used for reprocessing endoscopes, such as duodenoscopes, and endoscope accessories.

In the exemplary embodiment shown, the medical device processor 100 includes a basin assembly 200, process indicator isolation chamber 300, manifold 400, chemical dosing system 500, and a control system 600.

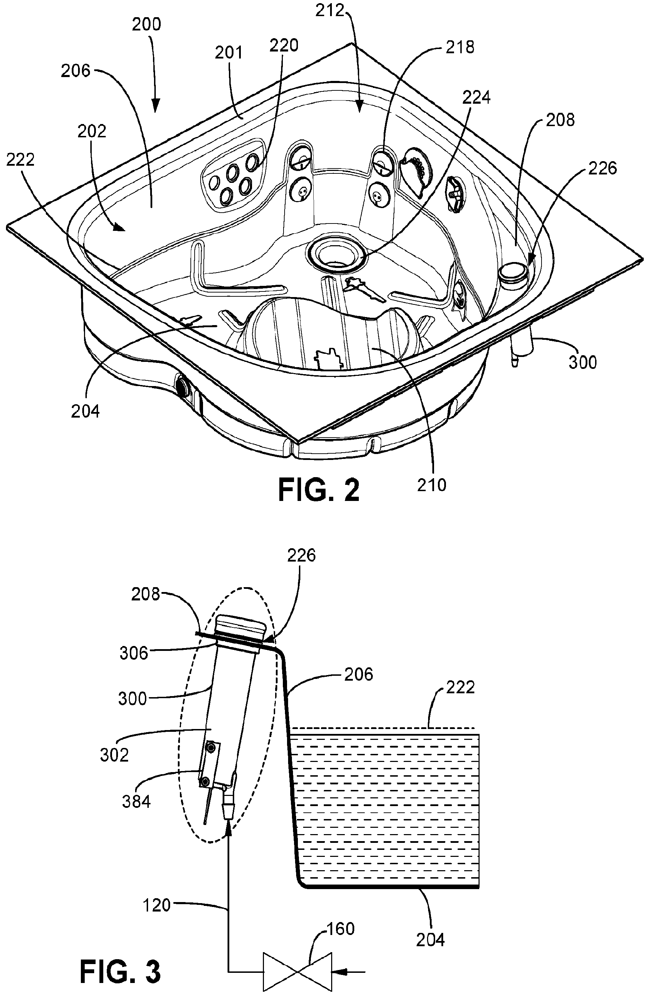

With additional reference to FIG. 2, the basin assembly 200 is configured to house the one or more medical devices for purposes of performing the cleaning and high-level disinfecting and/or sterilizing. The basin assembly 200 may have any suitable shape for retaining the one or more medical devices in a predetermined position during the process. In the example shown, the basin assembly 200 includes a tub 201 having an opening 202 (open top) for receiving a medical device for processing. The basin tub 201 includes a bottom surface 204 and one or more side walls 206. The one or more side walls extend from the bottom surface 204 and define a height/depth of the basin tub 201. In the embodiment shown, the basin includes a step surface 208. The step surface 208 is connected to the side wall 206. In the example shown, the basin tub 201 also includes a raised island 210 extending from the bottom surface 204.

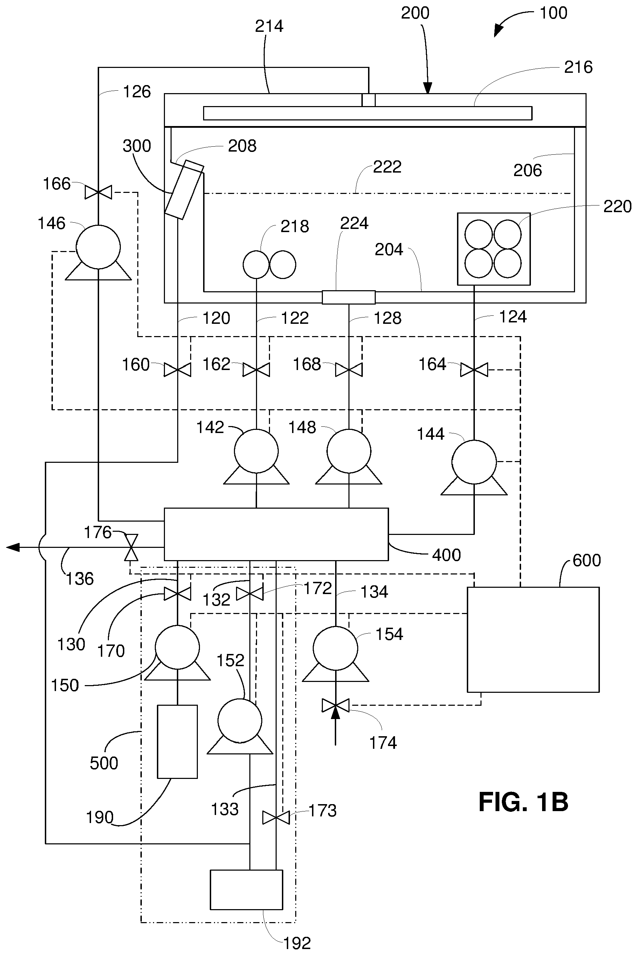

The basin tub 201 defines an interior volume 212 within which the one or more medical devices and liquid solution including detergent, high-level disinfectant solution, or chemical sterilant may be disposed during processing of the one or more medical devices. More specifically, the surfaces of the basin tub 201, including the bottom surface 204, one or more side walls 206, step surface 208, and raised island 210 collectively define the interior volume 212. A lid 214 (FIGS. 1A, 1B) or door may enclose the interior volume 212 of the basin tub 201 from the environment.

In some embodiments, the basin assembly 200 includes one or more spray bars 216 and/or one or more nozzles 218 for spraying the one or more medical devices with the liquid solution including detergent or high-level disinfectant solution and/or chemical sterilant during the cleaning process, high-level disinfecting process, and/or sterilizing process. In some embodiments, the basin assembly 200 includes one or more connectors 220 for connecting interior channels of a medical device to a source of detergent, high-level disinfectant solution, or chemical sterilant. In an example in which the medical device is an endoscope, the endoscope may be placed in the basin tub 201 and interior channels of the endoscope may be respectively connected to connectors 220. During the cleaning process, high-level disinfecting process, and/or sterilizing process, the outside surfaces and interior channels of the endoscope may be exposed to detergent, high-level disinfectant solution, and/or liquid chemical sterilant to clean surfaces of the endoscope and to kill microorganisms in or on the endoscope.

With additional reference to FIGS. 2 and 3, the basin assembly 200 includes a fill line 222. The fill line 222 designates the height of the at which the solution is filled in the basin tub 201 during processing. The basin tub 201 is configured such that not all internal surfaces are immersed. Parts of the side wall(s) of the basin tub 201 and/or other surfaces are above the fill line and not submerged when the basin is filled as part of the processing. Directed spray from the spray bar and/or nozzles is used to disinfect/sterilize non-immersed surfaces of the medical device being processed, as well as the non-immersed surfaces of the basin.

The basin assembly 200 includes a drain 224 for draining the solution including detergent, high-level disinfectant, or chemical sterilant from the basin tub 201. The liquid exiting the basin tub 201 via the drain 224 may in some embodiments be recirculated to the basin 200 (e.g., via manifold 400) during a given processing cycle. The liquid exiting the basin tub 201 via the drain 224 may also be discarded during or upon completion of a given cycle.

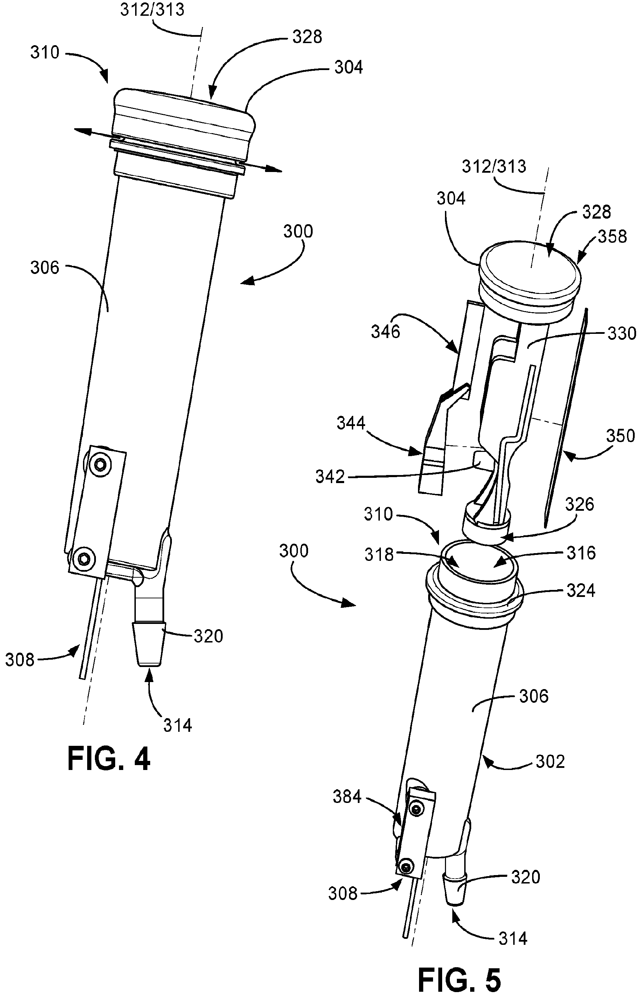

The basin tub 201 includes a through hole 226 located above the basin fill line 222. As shown, the isolation chamber 300 is disposed in and extends through the through hole 226. With additional reference to FIGS. 4-6, the isolation chamber 300 includes a housing 302 and a removable module 304.

The housing 302 extends through the through hole 226 and is mounted to the basin tub 201. A seal is formed between the outer surface 306 of the housing 302 and the basin tub 201 such that the interior volume of the basin tub is not in fluid communication with the environment via the through hole 226. In some embodiments, the housing 302 is adhered to the basin tub 201 via an adhesive. In other embodiments, the housing 302 is secured to the basin tub 201 via one or more mechanical fasteners (e.g., screws, clips, springs, and the like).

The housing 302 extends between a first end 308 and a second end 310 along a longitudinal axis 312. The first end 308 includes an inlet 314 and the second end 310 includes an opening 316. In the example shown, the housing 302 is tubular in shape and includes an interior volume 318 in fluid communication with the inlet 314 and the opening 316. As mounted to the basin tub 201, the first end 308 and inlet 314 of the housing are external to the internal volume of the basin tub 201, and the second end 310 and opening 316 of the housing are disposed in the interior of the basin tub 201.

The inlet 314 is connected to a supply line 120 (e.g., from the manifold 400) to supply liquid solution including high-level disinfectant solution and/or chemical sterilant to the interior volume 318 of the housing 302. In the embodiment shown, the inlet 314 at the first end 308 of the housing includes a fitting 320 to retain the supply line. The fitting 320 is exemplified as a barbed fitting. In other embodiments, the inlet 314 includes a different fitting, such as a thread, push-to-connect (e.g., SharkBite) connection, sanitary clamp, luer lock, and the like. In still other embodiments, the supply line may be retained on the inlet of the housing by an external component, such as a clamp, adhesive, and the like.

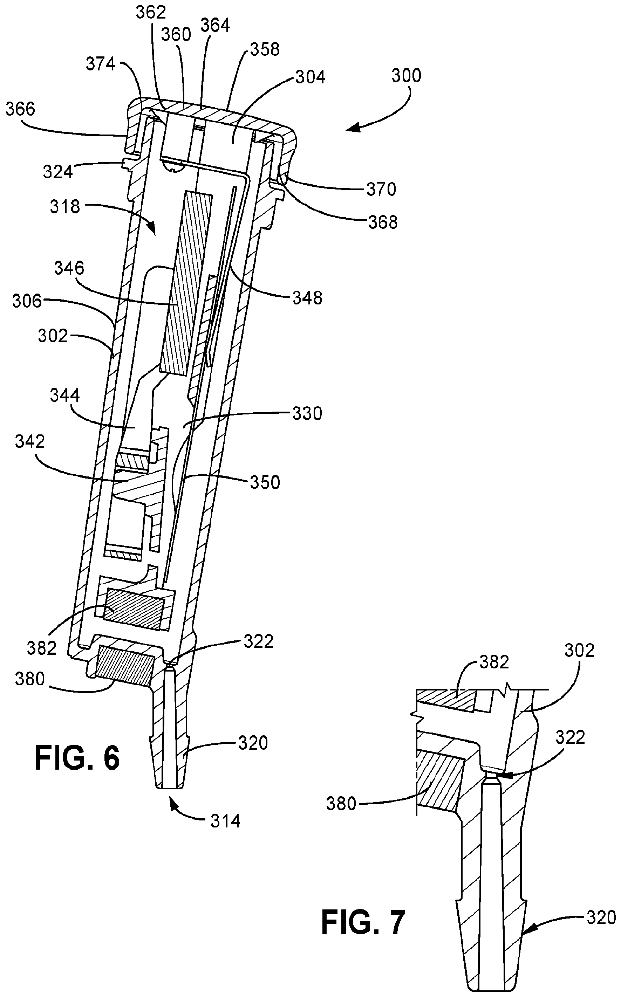

As shown in FIGS. 6 and 7, in some embodiments the inlet includes a restriction flow control orifice 322. The restriction flow control orifice 322 restricts and therefore throttles flowrate into and through the interior volume 318 of the housing 302. This can reduce the risk of microbial wash off on spore test strips and biological indicators, and can improve chemical indicator reliability. In other embodiments, the inlet flow control orifice 322 is not present. Flow restriction/throttling can be controlled by control of the supply of the liquid solution from the manifold 400. As an example, the flow can be controlled by control of the pump 140 and/or valve 160 connected to the supply line 120. A sensor (not shown), such as a flow sensor and/or pressure sensor, can be located in the supply line proximate the inlet to monitor the flowrate and/or pressure at which the liquid solution is supplied. In still other embodiments, a combination of the restriction flow control orifice 322 and the control of the supply of the liquid solution from the manifold 400 via the controller 600 is implemented.

The second end 310 of the housing 302 includes a flange 324 that radially extends from the outer surface 306 of the housing. As described below, the flange 324 acts in concert with the shielding cap of the module to create a tortuous outlet passage (flow path) that prevents spray from a spray bar and/or nozzles from entering the interior volume 318 of the housing via the second end 310.

The removable module 304 is removably disposed in the interior volume 318 of the housing. The removable module 304 extends along a longitudinal axis 313 between a first end 326 and a second end 328. When the removable module 304 is inserted in the housing 302, the longitudinal axis 313 of the removable module 304 is parallel to the longitudinal axis 312 of the housing 302.

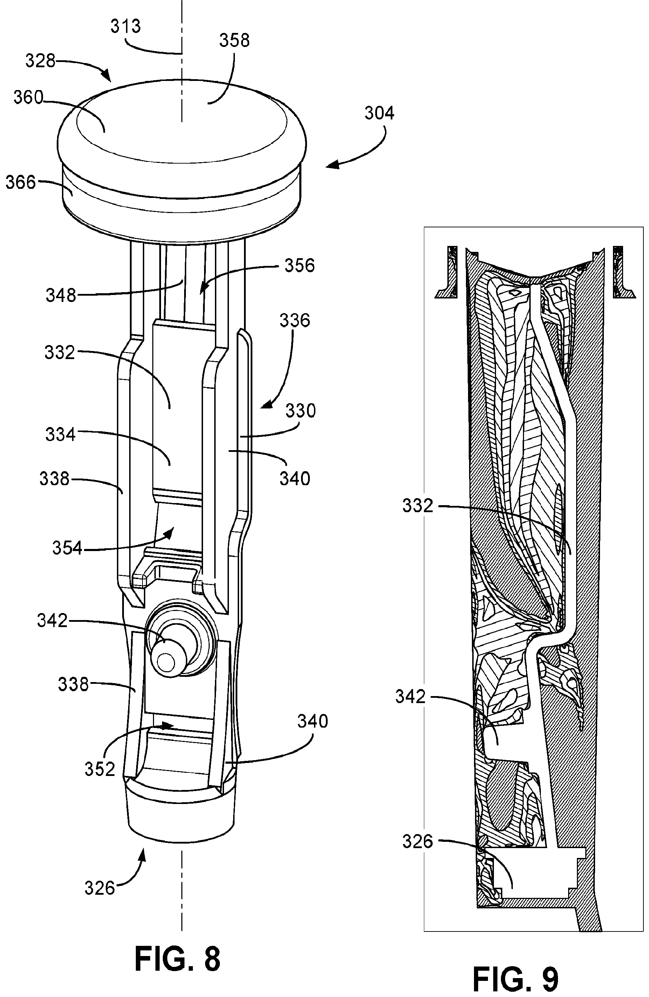

With additional reference to FIG. 8, the module 304 includes a frame 330 that extends along the longitudinal axis 313 between the first end 326 and the second end 328. The frame 330 includes a main body 332 having opposed major surfaces 334, 336. Side rails 338, 340 extend perpendicular to the main body 332 and along the longitudinal axis 313. The side rails 338, 340 extend from the major surface 334 of the main body 332 such that a channel is formed by the side rails and the main body. Similarly, the side rails extend from the major surface 336 of the main body 332 such that a channel is formed by the side rails and the main body. In some embodiments, the arrangement of the side rails 338, 340 help to direct fluid flow in the vicinity of the one or more process indicators attached to the removable module 304.

The module 304 includes a process indicator mount 342 extending from the major surface 334 of the main body 332. In the example shown, the process indicator mount 342 is a clip mounting post configured to mount a clip 344 holding a process indicator 346. In some embodiments, the process indicator mount 342 is for clips used to hold spore test strips, biological indicators, or other process indicators that cannot be directly handled. The clip 344 can be removably attached to the clip mounting post 342 for use in connection with a cleaning and high-level disinfecting and/or sterilizing process. In other embodiments, the process indicator mount 342 includes a clip fixed to the main body to hold a process indicator 346.

In the example shown, the module 304 further includes another process indicator mount 348 embodied as a spring clip. In the example shown, the spring clip 348 is attached to the shielding cap of the module and is shaped to retain a process indicator 350 against the major surface 336 of the main body 332 opposite the side from which the process indicator mount extends 342. The spring clip 348 can maintain the process indicator 350 against the major surface 336 via frictional force applied by the spring clip 348. In some embodiments, the spring clip 348 is a mounting clip for holding a chemical indicator strip to the module.

It will be appreciated that while the embodiment of the module shown in the figures includes two mounts (process indicator mount 342 and spring clip 348), in other embodiments only one of the mounts is included. In other embodiments, the mounts include two spring clips. In other embodiments, the mounts include two clip mounting posts. In still other embodiments, more than the two mounts can be included. In still other embodiments, the frame may have one or more mounts that are different from the process indicator mount and from the spring clip. For example, the module may have a basket or cage for containing process indicators. In still other embodiments, the process indicator may include no mounting provisions, and the indicators are secured using rubber bands, adhesive, tape, etc.

The main body 332 of the frame 330 is curved as viewed in the plane of the major surfaces 334, 336. This allows, for example, for the clip 344 mounted to the clip indicator mount 342 to be arranged in a manner such that the process indicator 346 is placed in an appropriate position when mounted. In the example shown, the main body 332 of the frame is curved such that the process indicator 346 is proximate the center of the housing interior volume 318 as viewed along the longitudinal axis 313.

The frame 330 includes cutout regions 352, 354, 356 that promote more uniform flow velocity inside of the housing and around the module. This can reduce flow stagnation and improve contact of the process indicator with the flowing liquid solution. The cutout regions 352, 354, 356 may improve flow via where the process indicator is retained by the frame, which may improve the reliability of the reading. FIG. 9 shows CFD flow velocity magnitude contours. As shown, the flow is present through the frame in the vicinity of the location of the process indicator when installed.

A shielding cap 358 is provided at the second end 326 of the removable module. The shielding cap 358 includes a top portion 360 having an inner surface 362 and an outer surface 364, and a side portion 366 extending from the top portion 360 and having an inner surface 368 and an outer surface 370. In the embodiment shown, the inner and outer surfaces 362, 364 of the top portion 360 are normal to the longitudinal axis 313 and the inner and outer surfaces 368, 370 of the side portion 366 extend from the top portion 360 in a direction parallel to the longitudinal axis 313.

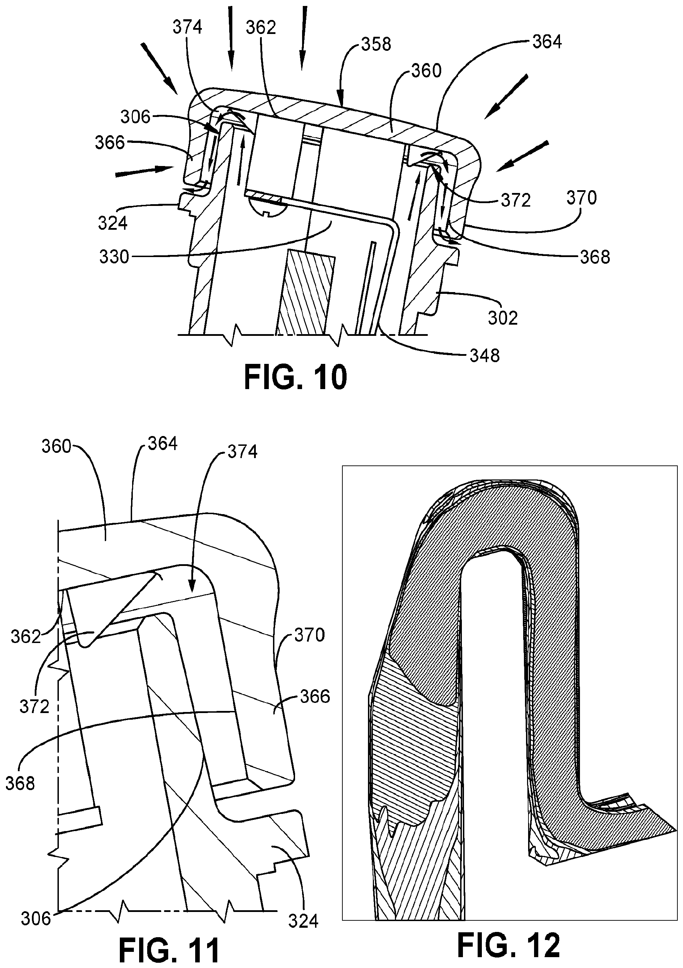

The shielding cap 358 includes offset gussets 372 extending from the inner surface 362 of the top portion 360 to maintain a desired gap between the second end 310 of the housing 302 and the shielding cap 358. With additional reference to FIG. 10, this allows for the fluid connection between the interior volume 318 of the housing 302 and the interior volume of the basin tub 201 to be maintained. Fluid can pass from the housing 302 to the basin tub 201 via the tortuous outlet passage (flow path) 374 defined by the inner surfaces 362, 368 of the shielding cap 358 and the outer surface 306 and flange 324 of the housing 302. When fluid is passed through the housing from the inlet 314, flow from the inlet can travel through the interior volume 318 of the housing 302, and out through the tortuous outlet passage 374, allowing fluid to exit. The tortuous outlet passage 374 also prevents basin spray from entering the isolation chamber assembly from the basin tub 201.

The tortuous outlet passage 374 created by the shielding cap 358 and flange 324 is configured to minimize stagnant flow regions along the tortuous outlet passage. This is achieved by eliminating any drastic expansions in cross-sectional area which produce flow eddies where air bubbles can become trapped. A cross-sectional view of the tortuous outlet passage 374 is shown in FIG. 11. FIG. 12 shows a CFD flow velocity contour in the tortuous outlet passage. As shown, the design provides a near constant cross-sectional area, which minimizes flow eddy regions. This ensures that all surfaces on the underside of the module receive sterilant contact, thereby improving the sterilization efficacy of the processor.

With continued reference to FIG. 6, the housing 302 includes a magnet 380 proximate the first end 308. The removable module 304 also includes a magnet 382 at its first end 326. The magnets 380, 382 work in concert to retain the module in the housing via magnetic force. In some embodiments, the isolation chamber assembly includes a sensor 384 that indicates that the module is in place in the housing. In some embodiments, the sensor 384 is a reed switch. The reed switch is mounted to the housing and is used by the controller 600 to verify whether the module has been re-inserted inside of the housing after the user loads the process indicators. The reed switch performs this function by detecting the magnetic field from the magnet 382 on the module 304. With this controller feedback, the controller can prevent the user from starting a processing cycle without re-inserting the module, and/or can provide context-specific instructions or animations for the process indicators on the display.

It will be appreciated that in other embodiments, the removable module 304 is maintained in the housing by a different configuration. For example, the housing 302 may include a threaded connector (not shown) at the bottom of the housing proximate the first end 314, and the removable module may include corresponding threaded connector (not shown) at its first end 326. The removable module may be threaded onto and maintained in the housing by the corresponding threaded connectors. It will also be appreciated that in some embodiments, a different switch is used to determine whether the removable module is provided in the housing. For example, a contact switch may be provided at the bottom of the housing proximate the first end, and the switch can be depressed upon insertion of the removable module. In other embodiments, no switch is provided.

With continued reference to FIG. 1A, in the exemplary embodiment shown, the manifold 400 is connected to the basin via one or more supply lines 122, 124, 126 and one or more return lines 128. The manifold 400 is also connected to the process indicator isolation chamber via supply line 120. Pumps 140, 142, 144, 146, 148 and valves 160, 162, 164, 166, 168 are also located along the respective supply and return lines for purposes of directing fluid flow in accordance with a desired cycle. For example, supply line 126 connects the manifold 400 to the spray bar(s) 216. Pump 146 and/or valve 166 are used in the control of flow of the liquid to the spray bar(s) 216. Supply line 122 connects the manifold 400 to the nozzle(s) 218. Pump 142 and/or valve 162 are used in the control of flow of the liquid to the nozzle(s) 218. Supply line 124 connects the manifold 400 to the connector(s) 220. Pump 144 and/or valve 164 are used in the control of flow of the liquid to the connector(s) 220. Supply line 120 connects the manifold 400 to the process indicator isolation chamber 300. Pump 140 and/or valve 160 are used in the control of flow of the liquid to the process indicator isolation chamber 300. Supply line 128 connects the basin drain 224 to the manifold 400. Pump 148 and/or valve 168 are used in the control of flow of the liquid from the drain 224 to the manifold 400.

The manifold 400 is also connected to a water supply line, the chemical dosing system 500, and a drain. Supply line 134 connects the water source to the manifold 400. Pump 154 and/or valve 174 are used in the control of flow of supply water to the manifold 400. The manifold 400 may be connected to the chemical dosing system 500 for purposes of receiving the detergent, high-level disinfectant solution, and/or liquid chemical sterilant. The manifold 400 may be used in connection with circulating the detergent, high-level disinfectant solution, and/or liquid chemical sterilant during a cleaning process, high-level disinfecting process, and/or sterilizing process.

The chemical dosing system 500 includes a detergent source 190. A supply line 130 connects the detergent source 190 to the manifold 400. Pump 150 and/or valve 170 are used in the control of flow of the detergent from the detergent source 190 to the manifold 400. In some embodiments, valve 170 is a check valve.

The chemical dosing system 500 includes a high-level disinfectant solution source and/or liquid chemical sterilant source 192. The high-level disinfectant and/or liquid chemical sterilant will also be referred to herein as decontaminant. In some embodiments, the decontaminant is peracetic acid and/or glutaraldehyde. Supply lines 132 and 133 form a supply path between the high-level disinfectant solution source and/or liquid chemical sterilant source 192 and the manifold 400. The supply line 132 connects the detergent manifold 400 to the high-level disinfectant solution source and/or liquid chemical sterilant source 192, and pump 152 and/or valve 172 are used in the control of flow of fluid from the manifold 400 to the high-level disinfectant solution source and/or liquid chemical sterilant source 192. Fluid can flow from the high-level disinfectant solution source and/or liquid chemical sterilant source 192 to the manifold via supply line 133. Valve 173 may be used in the control of flow of fluid from the source 192 to the manifold 400. Although not specifically shown, in other embodiments, the system may instead include a supply line and pump and/or valve arrangement similar to that shown in connection with the detergent source 190 that connects the high-level disinfectant solution source and/or liquid chemical sterilant source 192 to the manifold.

FIG. 1B shows another exemplary embodiment of the medical device processor 100. The embodiment shown in FIG. 1B is similar to the embodiment shown in FIG. 1A, but the supply line 120 of the process indicator isolation chamber 300 connects process indicator isolation chamber 300 to the manifold 400 via the supply line 133 to the high-level disinfectant solution source and/or liquid chemical sterilant source 192. In such embodiments, the supply lines 120 and 133 may be collectively referred to as a supply line. Reference is made to the description set forth above with respect to FIG. 1A, as said features apply to FIG. 1B and will not be described in detail for the sake of brevity.

It will be appreciated that in other embodiments, the medical device processor 100 may have any suitable arrangement of valves and pumps to effectuate the flow of liquid among the basin assembly 200 and manifold 400 during a given cycle. As such, any of the pumps and any of the valves may be omitted from the system. For example, in one embodiment, valves 162, 164, 166, and 168, and pumps 140, 142, 152, and 154 may be omitted from the medical device processor.

With continued reference to FIGS. 1A and 1B, the overall operation of the medical device processor 100, including the one or more pumps and valves may be controlled by control system 600. This control may effectuate, for example, any suitable cleaning, high-level disinfecting, sterilizing, and/or rinsing process.

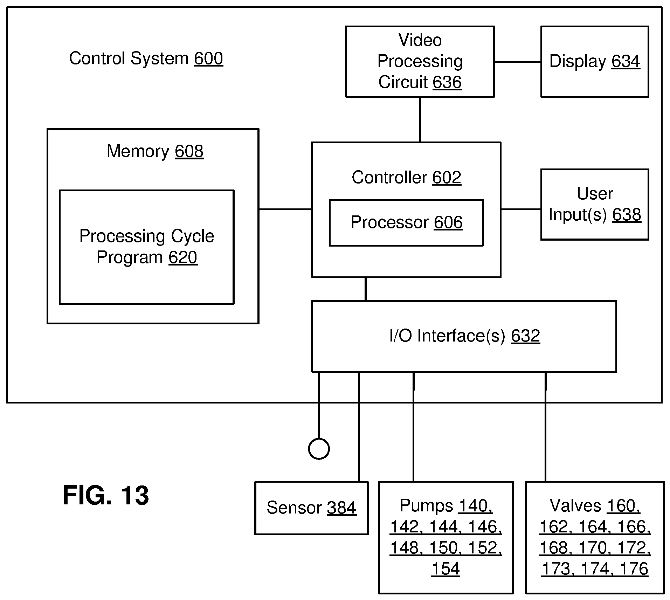

FIG. 13 shows an exemplary control system 600. Operation of the medical device processor, including the selective flow of fluid through the process indicator isolation chamber 300, is controlled by the control system 600. Components of the process indicator isolation chamber 300, such as the sensor 384, are connected to a controller 602 of the control system 600.

The controller 602 is configured to carry out overall control of the functions and operations of the control system 600. The controller 602 may include a processor 606, such as a central processing unit (CPU), microcontroller, or microprocessor. The processor 606 executes code stored in a memory (not shown) within the controller 602 and/or in a separate memory, such as the memory 608, in order to carry out operation of the various cleaning, disinfection/sterilization, and rinse cycles associated with a processing cycle; as well as the selective flow of fluid through the process indicator isolation chamber 300. As described above, the processor may also carry out overall operation of the medical device processor 100.

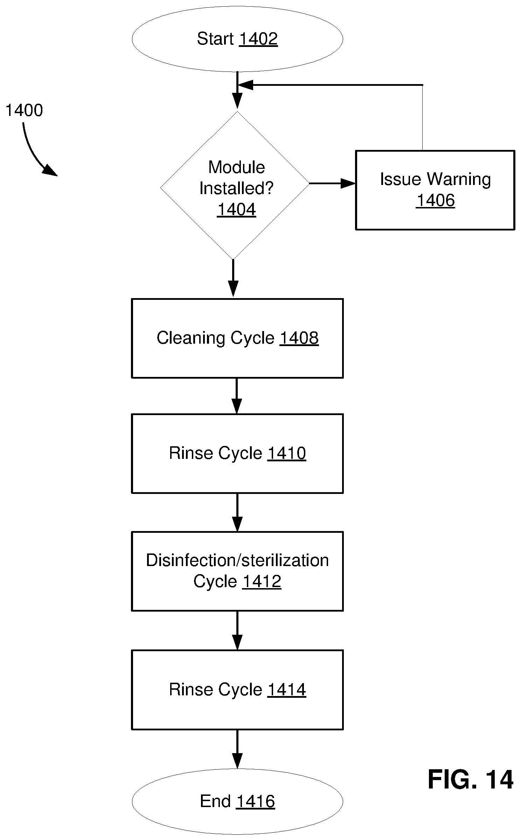

FIG. 13 shows an example in which a processing cycle program 620 is stored in the memory 608. This program may be embodied in the form of executable logic routines (e.g., lines of code, software programs, etc.) that are stored on a non-transitory computer readable medium (e.g., the memory 608) and executed by the controller 602 (e.g., using the processor 606). The processing cycle program 620 may be executed by the controller to control operation of the process cycle and implement a cleaning process and a high-level disinfecting process and/or sterilizing process (e.g., conducting a process cycle as described below with respect to FIG. 14).

For example, the processing cycle program 620 may be executed by the controller to prevent flow of detergent solution through the process indicator isolation chamber 300 during the cleaning cycle during which a detergent solution is used; and to permit flow of disinfectant and/or sterilization solution to flow through the process indicator isolation chamber 300 during the disinfection and/or sterilization process.

The processing cycle program 620 may be executed by the controller to control overall operation of the medical device processor to implement a cleaning process, high-level disinfecting process, and/or sterilizing process. For example, the processing cycle program 620 may be executed by the controller to execute a cleaning cycle in which the chemical dosing system delivers a dose of liquid for the cycle. As another example, the processing cycle program 620 may be executed by the controller to execute a high-level disinfection cycle in which the chemical dosing system delivers a dose of liquid for the cycle. As another example, the processing cycle program 620 may be executed by the controller to execute a sterilization cycle in which the chemical dosing system delivers a dose of liquid for the cycle.

The memory 608 may be, for example, one or more of a buffer, a flash memory, a hard drive, a removable media, a volatile memory, a non-volatile memory, a random access memory (RAM), or other suitable device. In a typical arrangement, the memory 608 may include a non-volatile memory for long term data storage and a volatile memory that functions as system memory for the controller 602. The memory 608 may exchange data with the controller 602 over a data bus. Accompanying control lines and an address bus between the memory 408 and the controller 602 also may be present. The memory 608 is considered a non-transitory computer readable medium.

The control system 600 may further include one or more input/output (I/O) interface(s) 632. The I/O interface(s) 632 may be in the form of one or more electrical connectors and may connect the controller 602 to one or more sensors, pumps, motors, valves, or other components. For example, as shown, the I/O interface(s) 632 is connected to the sensor 384 and the controller 602 may receive and process a signal received from the sensor via the I/O interface. As also shown, the I/O interface(s) 632 connect the controller to one or more of the pumps 140, 142, 144, 146, 148, 150, 152, 154, and one or more of the valves 160, 162, 164, 166, 168, 170, 172, 173, 174, 176 of the system 100, and the controller 602 may control operation of one or more of the pumps and valves.

The control system 600 may include a display 634. In some embodiments, the display 634 can display information such as the state/connection status of the process indicator isolation chamber 300. The display 634 may be a lighted display (e.g., a backlit liquid-crystal display (LCD) or organic light-emitting diode (OLED) display). The display 634 may be coupled to the controller 602 by a video processing circuit 636 that converts image and/or video data to an image and/or video signal used to drive the display 634. The video processing circuit 636 may include any appropriate buffers, decoders, video data processors and so forth. The control system 600 may include one or more user inputs 638 for receiving user input for controlling operation of the control system 600. Exemplary user inputs 638 include, but are not limited to, a touch input that overlays the display 634 for touch screen functionality, one or more buttons such as those included on the handle or in a different location, and so forth. The one or more user inputs 638 may, for example, allow a user to confirm that user action has been taken in response a warning or prompt issued and displayed on the display.

FIG. 14 is a flowchart showing an exemplary processing cycle. This process may be executed, for example, in a processing cycle in which both a cleaning cycle and a disinfection and/or sterilization cycle are conducted. The controller 600 may control the medical device processor to effectuate the processing cycle. It will be appreciated that while control is described with respect to the exemplary medical device processor 100 shown in the figures, in other embodiments the medical device processor 100 may have any suitable arrangement of valves and pumps to effectuate the flow of liquid among the basin assembly 200 and manifold 400 during a given cycle, and the controller 600 may control the valves and pumps in an appropriate manner to effectuate the cycle.

In advance of the process, a user may place one or medical devices to be processed in the basin. The medical device can be hooked up to one or more connections and arranged in a predetermined manner for processing. The user may also prepare the process indicator isolation chamber by removing the module from the housing, inserting and/or replacing the one or more process indicators on the module, and reinstalling the module in the housing.

At step 1402, a process cycle is stated. This may be done, for example, by receiving a command from a user via the user inputs 638. At step 1404, it is determined whether the process indicator isolation chamber is installed. If NO, then at step 1406 a warning is issued. The warning may be, for example displayed on the display 634. If YES, then processing proceeds to step 1408.

At step 1408, a cleaning cycle is conducted. During the cleaning cycle, water and detergent are introduced and mixed, and the resultant detergent containing solution is cycled through the basin and manifold. In the exemplary embodiment, the controller 600 controls pump 154 and/or valve 174 to introduce water, and controls pump 150 and/or valve 170 of the chemical dosing system 500 to introduce a predetermined amount of detergent. The cleaning cycle is conducted in a manner that the detergent containing solution is cycled through the basin and manifold for a predetermined amount of time by introducing the solution from the manifold to the basin via the spray bar(s) and nozzle(s), by injecting the solution into the channel(s) of the medical device via the connector(s), and by draining the solution from the basin back to the manifold. During this process, the valve(s) 160, 132, and/or pump(s) 140, 152 are controlled such that the solution is not introduced into the process indicator isolation chamber. Chemical indicator test strips, spore test strips, or biological indicators are intended to verify disinfection or sterilization efficacy, either by monitoring solution concentration or microbial elimination. These process indicators can only be exposed to disinfectant/sterilant solutions and rinse water. They cannot be exposed to any cleaning detergents or enzymes, as this would alter the accuracy or reliability of the results. Accordingly, the process keeps the chemical indicator test strips, spore test strips, and/or biological indicators intended to verify disinfection or sterilization efficacy isolated from the detergent solution used in the cleaning cycle. At the end of the cleaning cycle, the spray bar(s), nozzle(s), and connector(s) are deactivated and the solution is drained from the basin and removed from the system via drain line.

At step 1410, a rinse cycle is conducted. During the rinse cycle, water is introduced and cycled through the basin and manifold. In the exemplary embodiment, the controller controls pump 154 and/or valve 174 to introduce water. The rinse cycle is conducted in a manner that the water is cycled through the basin and manifold for a predetermined amount of time by introducing the water from the manifold to the basin via the spray bar(s) and nozzle(s), by injecting the water into the channel(s) of the medical device via the connector(s), and by draining the water from the basin back to the manifold. During this process, the valve(s) 160, 132, and/or pump(s) 140, 152 are controlled such that the rinse water is not introduced into the process indicator isolation chamber. At the end of the rinse cycle, the spray bar(s), nozzle(s), and connector(s) are deactivated and the water is drained from the basin and removed from the system via drain line.

At step 1412, the disinfection/sterilization cycle is conducted. During the disinfection/sterilization cycle, disinfectant/sterilant containing solution is cycled through the basin and manifold. In the exemplary embodiment, the controller 600 controls pump 154 and/or valve 174 to introduce water and controls pump 152 and/or valves 172, 173 of the chemical dosing system 500 to introduce a predetermined amount of disinfectant/sterilant. The disinfection/sterilization cycle is conducted in a manner that the disinfectant/sterilant containing solution is cycled through the basin and manifold for a predetermined amount of time by introducing the solution from the manifold to the basin via the spray bar(s) and nozzle(s), by injecting the solution into the channel(s) of the medical device via the connector(s), and by draining the solution from the basin back to the manifold. During this process, the valve(s) 160, 132 and/or pump(s) 140, 152 are controlled such that the solution is introduced into the process indicator isolation chamber 300. The chemical indicator test strips, spore test strips, and/or biological indicators included in the process indicator isolation chamber are brought into contact with the solution to verify disinfection or sterilization efficacy. At the end of the disinfection/sterilization cycle, the spray bar(s), nozzle(s), and connector(s) are deactivated and the solution is drained from the basin and removed from the system via drain line.

At step 1414, a rinse cycle is conducted. During the rinse cycle, water is introduced and cycled through the basin and manifold. In the exemplary embodiment, the controller controls pump 154 and/or valve 174 to introduce water. The rinse cycle is conducted in a manner that the water is cycled through the basin and manifold for a predetermined amount of time by introducing the water from the manifold to the basin via the spray bar(s) and nozzle(s), by injecting the water into the channel(s) of the medical device via the connector(s), and by draining the water from the basin back to the manifold. At the end of the rinse cycle, the spray bar(s), nozzle(s), and connector(s) are deactivated and the water is drained from the basin and removed from the system via drain line. During this cycle, in some embodiments, the valve(s) 160, 132 and/or pump(s) 140, 152 are controlled such that the rinse water is introduced into the process indicator isolation chamber. In other embodiments, the valve(s) 160, 132 and/or pump(s) 160, 152 are controlled such that the solution is not introduced into the process indicator isolation chamber.

At step 1416, the process ends. Following the process, a user can remove the processed medical device(s) and can also remove the one or more process indicators to verify disinfection or sterilization efficacy.

It will be appreciated that the system and process as described herein can also be used in a passthrough (two-sided) configuration. By locating the process indicator isolation chamber inside of the basin, users can access the process indicators on both sides of the passthrough configuration device. A user can load a new process indicator when starting a cycle on the "dirty" side, then another user can unload and inspect the process indicators on the "clean" side when the cycle is complete.

## Claims

1. A process indicator isolation chamber (300), comprising:
a housing (302) having an interior volume, the housing (302) comprising:
an inlet (314) at a first end (308) of the housing (302);
an opening (316) at a second end (310) of the housing (302) opposite the first end (308);
a flange (324) extending radially outward from an outer surface (306) of the housing (302), proximate the second end (310); and
a removable module (304) comprising:
a frame (330) at least partially disposed in the interior volume of the housing, the removable module (304) comprising a first end (326) disposed in the interior volume of the housing (302);
a shielding cap (358) attached to the frame (330) at a second end (328) of the removable module (304) opposite the first end (326) of the removable module (304), the shielding cap (358) including a top portion (360) that covers the opening (316) of the housing (302) and a side portion (366) that covers a portion of the outer surface of the housing (302) between the flange (324) and the second end (310) of the housing (302); and
a process indicator mount (342,348) attached to the frame (330) and disposed in the interior volume of the housing (302), the process indicator mount (342,348) configured to hold at least one test indicator strip in a fluid path between the inlet (314) and the opening (316);
wherein a tortuous outlet passage (374) is formed between the shielding cap (358) and the outer surface (306) of the housing (302).

2. The process indicator isolation chamber of claim 1, further comprising:
a first magnet (380) attached to the first end (308) of the frame (330); and
a second magnet (382) attached to the housing (302) proximate the second end (310) of the housing (302),
wherein the first magnet (380) and the second magnet (382) interact to retain the module (304) in the housing (302) via magnetic force.

3. The process indicator isolation chamber of any one of claims 1-2, further comprising a switch (384) configured to detect the insertion state of the removable module (304) in the housing (302).

4. The process indicator isolation chamber of claim 3, wherein the switch (384) is a reed switch.

5. The process indicator isolation chamber of any one of claims 1-4, wherein the process indicator mount (342) comprises a clip mounting post configured to mount a clip (344) holding a process indicator (346).

6. The process indicator isolation chamber of any one of claims 1-4, wherein the process indicator mount (342) comprises a clip (344) configured to hold a process indicator (346).

7. The process indicator isolation chamber of any one of claims 1-4, wherein the process indicator mount (348) comprises a spring clip (348).

8. The process indicator isolation chamber of any one of claims 1-7, wherein the process indicator mount is a first process indicator mount (342), and the removable module (304) comprises a second process indicator mount (348) configured to hold an additional test indicator strip.

9. The process indicator isolation chamber of any one of claims 1-8, wherein the frame (330) comprises:
a main body (332) having opposed major surfaces (334,336); and
side rails (338,340) extending perpendicular to the main body (332), the side rails (338,340) and the main body (332) forming a channel, wherein the process indicator mount (342) is arranged to provide the process indicator at least partially in the channel.

10. The process indicator isolation chamber of claim 9, comprising one or more cut out regions (352,354,356) extending through the main body (332), the one or more cut out regions (352,354,356) configured to allow fluid flow through the main body (332).

11. The process indicator isolation chamber of any one of claims 1-10, wherein the shielding cap (358) comprises offset gussets (372) extending from the inner surface (362) of the top portion (360) to maintain a gap between the second end (310) of the housing (302) and the shielding cap (358).

12. A medical device processor (100), comprising:
a basin assembly (200) comprising a basin tub (201); and
the process indicator isolation chamber (300) of any one of claims 1-11 disposed in a through hole of the basin tub (201), the inlet of the process indicator isolation chamber (200) housing positioned outside the basin tub (201), the opening at the second end of the housing positioned inside the basin tub,
wherein the process indicator isolation chamber (300) is fluidly connected with the basin tub (201) such that fluid can flow through the isolation chamber (300) into the basin tub (201).

13. The medical device processor of claim 12, wherein:
wherein the basin assembly (200) further comprises:
a lid (214) configured to selectively cover the basin tub (201);
one or more connectors (220) in fluid communication with the internal volume of the basin tub (201) and configured to connect to a medical device processed in the medical device processor (100);
one or more spray bars (216) in fluid communication with the interior volume of the basin tub (201); and
a drain (224) in fluid communication with the interior volume of the basin tub (201),
wherein the tortuous outlet passage (374) of the process indicator isolation chamber (300) is in fluid communication with the interior volume of the basin tub (201).

14. The medical device processor of claim 13, further comprising:
a manifold (400); and
a supply line (124) connecting the manifold (400) to the one or more connectors (220);
a pump (144) and/or valve (164) to control flow of fluid to the one or more connectors (220);
a supply line (126) connecting the manifold (400) to the one or more spray bars (216);
a pump (146) and/or valve (166) to control flow of fluid to the one or more spray bars (216);
a supply line (120) connecting the manifold (400) to the process indicator isolation chamber (300);
a pump (140) and/or valve (162) to control flow of fluid to the process indicator isolation chamber (300);
a return line (128) connecting the drain (224) to the manifold (300); and
a pump (148) and/or valve (168) to control flow of fluid from the drain (224) of the basin assembly (200) to the manifold (300).

15. The medical device processor of claim 14, further comprising a controller (600) configured to control the respective valves and/or pumps of the supply lines and the pump and/or valve of the return line.

16. The medical device processor of claim 15, wherein the controller (600) is configured to execute a processing cycle program (620) stored on a memory (608), the processing cycle program (620) comprising:
a cleaning cycle in which a solution comprising detergent is cycled through the basin tub (201) and manifold (400) for a predetermined amount of time by introducing the solution comprising detergent from the manifold (400) to the basin tub (201) via the one or more spray bars (216) and the one or more connectors (220), and by draining the solution from the basin tub (201) back to the manifold (400), the pump (140) and/or valve (160) associated with the process indicator isolation chamber (300) being controlled by the controller (600) such that the solution comprising the detergent is not introduced into the process indicator isolation chamber (300); and
a decontamination cycle in which a solution comprising a decontaminant is cycled through the basin tub (201) and manifold (400) for a predetermined amount of time by introducing the solution comprising the decontaminant from the manifold (400) to the basin tub (201) via the one or more spray bars (216) and one or more connectors (220), and by draining the solution comprising the decontaminant from the basin tub (201) back to the manifold (400), the pump (140) and/or valve (160) associated with the process indicator isolation chamber (300) being controlled by the controller such that the solution comprising the decontaminant is introduced into the process indicator isolation chamber (300).

17. The medical device process of claim 16, wherein the decontaminant is a high-level disinfectant.

18. The medical device process of claim 16, wherein the decontaminant is a sterilant.

19. The medical device processor of claim 16, wherein the decontaminant is peracetic acid or glutaraldehyde.

20. A method of determining the efficacy of a disinfection process comprising the steps of:
performing a cleaning cycle in a medical device processor, the medical device processor (100) comprising:
a basin assembly (200) comprising a basin tub (201);
a lid (214) configured to selectively cover the basin tub (201);
one or more nozzles (218) in fluid communication with the internal volume of the basin tub (201);
one or more connectors (220) in fluid communication with the internal volume of the basin tub (201) and configured to connect to a medical device processed in the medical device processor (100);
one or more spray (216) bars in fluid communication with the interior volume of the basin tub (201); and
the process indicator isolation chamber (300) of any one of claims 1-11 disposed in a through hole of the basin tub (201), the inlet (314) of the process indicator isolation chamber housing (302) positioned outside the basin tub (201), the opening (316) at the second end (310) of the housing (302) positioned inside the basin tub (201), wherein the process indicator isolation chamber (300) is fluidly connected with the basin tub (201) such that fluid from the basin tub (201) can be selectively flowed through the isolation chamber (300),
wherein the cleaning cycle comprises introducing solution comprising detergent into to the basin tub (201) via the one or more spray bars (216), the one or more nozzles (218), and the one or more connectors (220), the solution comprising the detergent not being introduced into the process indicator isolation chamber (300) during the cleaning cycle; and
performing a decontamination cycle comprising introducing solution comprising decontaminant into to the basin tub (201) via the one or more spray bars (216), the one or more nozzles (218), and the one or more connectors (220), the solution comprising the decontaminant being introduced into the process indicator isolation chamber (300) during the decontamination cycle.

## Patentansprüche

1. Prozessindikator-Isolationskammer (300), umfassend:
ein Gehäuse (302) mit einem Innenvolumen, wobei das Gehäuse (302) Folgendes umfasst:
einen Einlass (314) an einem ersten Ende (308) des Gehäuses (302);
eine Öffnung (316) an einem zweiten Ende (310) des Gehäuses (302) gegenüber dem ersten Ende (308);
einen Flansch (324), der sich von einer Außenfläche (306) des Gehäuses (302) radial nach außen erstreckt, nahe dem zweiten Ende (310); und
ein entnehmbares Modul (304), umfassend:
einen Rahmen (330), der zumindest teilweise in dem Innenvolumen des Gehäuses angeordnet ist, wobei das entnehmbare Modul (304) ein erstes Ende (326) umfasst, das in dem Innenvolumen des Gehäuses (302) angeordnet ist;
eine Abschirmkappe (358), die an dem Rahmen (330) an einem zweiten Ende (328) des entnehmbaren Moduls (304) gegenüber dem ersten Ende (326) des entnehmbaren Moduls (304) befestigt ist, wobei die Abschirmkappe (358) einen oberen Abschnitt (360), der die Öffnung (316) des Gehäuses (302) abdeckt, und einen Seitenabschnitt (366) umfasst, der einen Abschnitt der Außenfläche des Gehäuses (302) zwischen dem Flansch (324) und dem zweiten Ende (310) des Gehäuses (302) abdeckt; und
eine Prozessindikatorhalterung (342,348), die an dem Rahmen (330) befestigt und in dem Innenvolumen des Gehäuses (302) angeordnet ist, wobei die Prozessindikatorhalterung (342,348) dazu ausgebildet ist, mindestens einen Testindikatorstreifen in einem Fluidweg zwischen dem Einlass (314) und der Öffnung (316) zu halten;
wobei zwischen der Abschirmkappe (358) und der Außenfläche (306) des Gehäuses (302) ein gewundener Auslasskanal (374) ausgebildet ist.

2. Prozessindikator-Isolationskammer nach Anspruch 1, ferner umfassend:
einen ersten Magneten (380), der an dem ersten Ende (308) des Rahmens (330) befestigt ist; und
einen zweiten Magneten (382), der an dem Gehäuse (302) nahe dem zweiten Ende (310) des Gehäuses (302) befestigt ist,
wobei der erste Magnet (380) und der zweite Magnet (382) zusammenwirken, um das Modul (304) mittels Magnetkraft in dem Gehäuse (302) zu halten.

3. Prozessindikator-Isolationskammer nach einem der Ansprüche 1-2, ferner umfassend einen Schalter (384), der dazu ausgebildet ist, den Einsetzzustand des entnehmbaren Moduls (304) in dem Gehäuse (302) zu erfassen.

4. Prozessindikator-Isolationskammer nach Anspruch 3, wobei der Schalter (384) ein Reedschalter ist.

5. Prozessindikator-Isolationskammer nach einem der Ansprüche 1-4, wobei die Prozessindikatorhalterung (342) einen Clip-Montagezapfen umfasst, der dazu ausgebildet ist, einen Clip (344) zu montieren, der einen Prozessindikator (346) hält.

6. Prozessindikator-Isolationskammer nach einem der Ansprüche 1-4, wobei die Prozessindikatorhalterung (342) einen Clip (344) umfasst, der dazu ausgebildet ist, einen Prozessindikator (346) zu halten.

7. Prozessindikator-Isolationskammer nach einem der Ansprüche 1-4, wobei die Prozessindikatorhalterung (348) einen Federclip (348) umfasst.

8. Prozessindikator-Isolationskammer nach einem der Ansprüche 1-7, wobei die Prozessindikatorhalterung eine erste Prozessindikatorhalterung (342) ist und das entnehmbare Modul (304) eine zweite Prozessindikatorhalterung (348) umfasst, die dazu ausgebildet ist, einen zusätzlichen Testindikatorstreifen zu halten.

9. Prozessindikator-Isolationskammer nach einem der Ansprüche 1-8, wobei der Rahmen (330) Folgendes umfasst:
einen Hauptkörper (332) mit gegenüberliegenden Hauptflächen (334,336); und
Seitenschienen (338,340), die sich senkrecht zu dem Hauptkörper (332) erstrecken, wobei die Seitenschienen (338,340) und der Hauptkörper (332) einen Kanal bilden, wobei die Prozessindikatorhalterung (342) dazu angeordnet ist, den Prozessindikator zumindest teilweise in dem Kanal bereitzustellen.

10. Prozessindikator-Isolationskammer nach Anspruch 9, umfassend einen oder mehrere Ausschnittbereiche (352,354,356), die sich durch den Hauptkörper (332) erstrecken, wobei der eine oder die mehreren Ausschnittbereiche (352,354,356) dazu ausgebildet sind, einen Fluidstrom durch den Hauptkörper (332) zu ermöglichen.

11. Prozessindikator-Isolationskammer nach einem der Ansprüche 1-10, wobei die Abschirmkappe (358) versetzte Verstärkungsrippen (372) umfasst, die sich von der Innenfläche (362) des oberen Abschnitts (360) erstrecken, um einen Spalt zwischen dem zweiten Ende (310) des Gehäuses (302) und der Abschirmkappe (358) aufrechtzuerhalten.

12. Aufbereitungsvorrichtung für medizinische Vorrichtungen (100), umfassend:
eine Beckenbaugruppe (200), die eine Beckenwanne (201) umfasst; und
die Prozessindikator-Isolationskammer (300) nach einem der Ansprüche 1-11, die in einem Durchgangsloch der Beckenwanne (201) angeordnet ist, wobei der Einlass des Gehäuses der Prozessindikator-Isolationskammer (200) außerhalb der Beckenwanne (201) angeordnet ist, wobei die Öffnung an dem zweiten Ende des Gehäuses innerhalb der Beckenwanne angeordnet ist,
wobei die Prozessindikator-Isolationskammer (300) fluidisch mit der Beckenwanne (201) verbunden ist, sodass Fluid durch die Isolationskammer (300) in die Beckenwanne (201) strömen kann.

13. Aufbereitungsvorrichtung für medizinische Vorrichtungen nach Anspruch 12, wobei:
wobei die Beckenbaugruppe (200) ferner Folgendes umfasst:
einen Deckel (214), der dazu ausgebildet ist, die Beckenwanne (201) selektiv abzudecken;
einen oder mehrere Anschlüsse (220), die in Fluidverbindung mit dem Innenvolumen der Beckenwanne (201) stehen und dazu ausgebildet sind, mit einer medizinischen Vorrichtung verbunden zu werden, die in der Aufbereitungsvorrichtung für medizinische Vorrichtungen (100) aufbereitet wird;
ein oder mehrere Sprührohre (216), die in Fluidverbindung mit dem Innenvolumen der Beckenwanne (201) stehen; und
einen Ablauf (224), der in Fluidverbindung mit dem Innenvolumen der Beckenwanne (201) steht,
wobei der gewundene Auslasskanal (374) der Prozessindikator-Isolationskammer (300) in Fluidverbindung mit dem Innenvolumen der Beckenwanne (201) steht.

14. Aufbereitungsvorrichtung für medizinische Vorrichtungen nach Anspruch 13, ferner umfassend:
einen Verteiler (400); und
eine Zuleitung (124), die den Verteiler (400) mit dem einen oder den mehreren Anschlüssen (220) verbindet;
eine Pumpe (144) und/oder ein Ventil (164) zum Steuern des Fluidstroms zu dem einen oder den mehreren Anschlüssen (220);
eine Zuleitung (126), die den Verteiler (400) mit dem einen oder den mehreren Sprührohren (216) verbindet;
eine Pumpe (146) und/oder ein Ventil (166) zum Steuern des Fluidstroms zu dem einen oder den mehreren Sprührohren (216);
eine Zuleitung (120), die den Verteiler (400) mit der Prozessindikator-Isolationskammer (300) verbindet;
eine Pumpe (140) und/oder ein Ventil (162) zum Steuern des Fluidstroms zu der Prozessindikator-Isolationskammer (300);
eine Rücklaufleitung (128), die den Ablauf (224) mit dem Verteiler (300) verbindet; und
eine Pumpe (148) und/oder ein Ventil (168) zum Steuern des Fluidstroms von dem Ablauf (224) der Beckenbaugruppe (200) zu dem Verteiler (300).

15. Aufbereitungsvorrichtung für medizinische Vorrichtungen nach Anspruch 14, ferner umfassend eine Steuerung (600), die dazu ausgebildet ist, die jeweiligen Ventile und/oder Pumpen der Zuleitungen und die Pumpe und/oder das Ventil der Rücklaufleitung zu steuern.

16. Aufbereitungsvorrichtung für medizinische Vorrichtungen nach Anspruch 15, wobei die Steuerung (600) dazu ausgebildet ist, ein auf einem Speicher (608) gespeichertes Aufbereitungszyklusprogramm (620) auszuführen, wobei das Aufbereitungszyklusprogramm (620) Folgendes umfasst:
einen Reinigungszyklus, in dem eine Reinigungsmittel umfassende Lösung für eine vorbestimmte Zeitdauer durch die Beckenwanne (201) und den Verteiler (400) zirkuliert wird, indem die Reinigungsmittel umfassende Lösung von dem Verteiler (400) über das eine oder die mehreren Sprührohre (216) und den einen oder die mehreren Anschlüsse (220) in die Beckenwanne (201) eingeleitet wird und indem die Lösung aus der Beckenwanne (201) zurück zu dem Verteiler (400) abgelassen wird, wobei die der Prozessindikator-Isolationskammer (300) zugeordnete Pumpe (140) und/oder das zugeordnete Ventil (160) derart von der Steuerung (600) gesteuert werden, dass die das Reinigungsmittel umfassende Lösung nicht in die Prozessindikator-Isolationskammer (300) eingeleitet wird; und
einen Dekontaminationszyklus, in dem eine ein Dekontaminationsmittel umfassende Lösung für eine vorbestimmte Zeitdauer durch die Beckenwanne (201) und den Verteiler (400) zirkuliert wird, indem die das Dekontaminationsmittel umfassende Lösung von dem Verteiler (400) über das eine oder die mehreren Sprührohre (216) und einen oder mehrere Anschlüsse (220) in die Beckenwanne (201) eingeleitet wird und indem die das Dekontaminationsmittel umfassende Lösung aus der Beckenwanne (201) zurück zu dem Verteiler (400) abgelassen wird, wobei die der Prozessindikator-Isolationskammer (300) zugeordnete Pumpe (140) und/oder das zugeordnete Ventil (160) derart von der Steuerung gesteuert werden, dass die das Dekontaminationsmittel umfassende Lösung in die Prozessindikator-Isolationskammer (300) eingeleitet wird.

17. Aufbereitung für medizinische Vorrichtungen nach Anspruch 16, wobei das Dekontaminationsmittel ein High-Level-Desinfektionsmittel ist.

18. Aufbereitung für medizinische Vorrichtungen nach Anspruch 16, wobei das Dekontaminationsmittel ein Sterilisationsmittel ist.

19. Aufbereitungsvorrichtung für medizinische Vorrichtungen nach Anspruch 16, wobei das Dekontaminationsmittel Peressigsäure oder Glutaraldehyd ist.

20. Verfahren zum Bestimmen der Wirksamkeit eines Desinfektionsprozesses, umfassend die folgenden Schritte:
Durchführen eines Reinigungszyklus in einer Aufbereitungsvorrichtung für medizinische Vorrichtungen, wobei die Aufbereitungsvorrichtung für medizinische Vorrichtungen (100) Folgendes umfasst:
eine Beckenbaugruppe (200), die eine Beckenwanne (201) umfasst;
einen Deckel (214), der dazu ausgebildet ist, die Beckenwanne (201) selektiv abzudecken;
eine oder mehrere Düsen (218), die in Fluidverbindung mit dem Innenvolumen der Beckenwanne (201) stehen;
einen oder mehrere Anschlüsse (220), die in Fluidverbindung mit dem Innenvolumen der Beckenwanne (201) stehen und dazu ausgebildet sind, mit einer medizinischen Vorrichtung verbunden zu werden, die in der Aufbereitungsvorrichtung für medizinische Vorrichtungen (100) aufbereitet wird;
ein oder mehrere Sprührohre (216), die in Fluidverbindung mit dem Innenvolumen der Beckenwanne (201) stehen; und
die Prozessindikator-Isolationskammer (300) nach einem der Ansprüche 1-11, die in einem Durchgangsloch der Beckenwanne (201) angeordnet ist, wobei der Einlass (314) des Gehäuses (302) der Prozessindikator-Isolationskammer außerhalb der Beckenwanne (201) positioniert ist, wobei die Öffnung (316) an dem zweiten Ende (310) des Gehäuses (302) innerhalb der Beckenwanne (201) positioniert ist, wobei die Prozessindikator-Isolationskammer (300) fluidisch mit der Beckenwanne (201) verbunden ist, sodass Fluid aus der Beckenwanne (201) selektiv durch die Isolationskammer (300) strömen kann,
wobei der Reinigungszyklus das Einleiten einer Reinigungsmittel umfassenden Lösung in die Beckenwanne (201) über das eine oder die mehreren Sprührohre (216), die eine oder die mehreren Düsen (218) und den einen oder die mehreren Anschlüsse (220) umfasst, wobei die das Reinigungsmittel umfassende Lösung während des Reinigungszyklus nicht in die Prozessindikator-Isolationskammer (300) eingeleitet wird; und Durchführen eines Dekontaminationszyklus, umfassend das Einleiten einer Dekontaminationsmittel umfassenden Lösung in die Beckenwanne (201) über das eine oder die mehreren Sprührohre (216), die eine oder die mehreren Düsen (218) und den einen oder die mehreren Anschlüsse (220), wobei die das Dekontaminationsmittel umfassende Lösung während des Dekontaminationszyklus in die Prozessindikator-Isolationskammer (300) eingeleitet wird.

## Revendications

1. Chambre d'isolation d'indicateur de processus (300), comprenant :
un boîtier (302) ayant un volume intérieur, le boîtier (302) comprenant :
une entrée (314) à une première extrémité (308) du boîtier (302) ;
une ouverture (316) à une seconde extrémité (310) du boîtier (302) à l'opposé de la première extrémité (308) ;
une bride (324) s'étendant radialement vers l'extérieur à partir d'une surface extérieure (306) du boîtier (302), à proximité de la seconde extrémité (310) ; et
un module amovible (304) comprenant :
un cadre (330) disposé au moins partiellement dans le volume intérieur du boîtier, le module amovible (304) comprenant une première extrémité (326) disposée dans le volume intérieur du boîtier (302) ;
un capuchon de protection (358) fixé au cadre (330) à une seconde extrémité (328) du module amovible (304) à l'opposé de la première extrémité (326) du module amovible (304), le capuchon de protection (358) comportant une partie supérieure (360) qui recouvre l'ouverture (316) du boîtier (302) et une partie latérale (366) qui recouvre une partie de la surface extérieure du boîtier (302) entre la bride (324) et la seconde extrémité (310) du boîtier (302) ; et
un support d'indicateur de processus (342, 348) fixé au cadre (330) et disposé dans le volume intérieur du boîtier (302), le support d'indicateur de processus (342, 348) étant configuré pour maintenir au moins une bande indicatrice de test dans un trajet de fluide entre l'entrée (314) et l'ouverture (316) ;
dans laquelle un passage de sortie tortueux (374) est formé entre le capuchon de protection (358) et la surface extérieure (306) du boîtier (302).

2. Chambre d'isolation d'indicateur de processus selon la revendication 1, comprenant en outre :
un premier aimant (380) fixé à la première extrémité (308) du cadre (330) ; et
un second aimant (382) fixé au boîtier (302) à proximité de la seconde extrémité (310) du boîtier (302),
dans laquelle le premier aimant (380) et le second aimant (382) interagissent pour retenir le module (304) dans le boîtier (302) par l'intermédiaire d'une force magnétique.

3. Chambre d'isolation d'indicateur de processus selon l'une quelconque des revendications 1 à 2, comprenant en outre un commutateur (384) configuré pour détecter l'état d'insertion du module amovible (304) dans le boîtier (302).

4. Chambre d'isolation d'indicateur de processus selon la revendication 3, dans laquelle le commutateur (384) est un commutateur à lames.

5. Chambre d'isolation d'indicateur de processus selon l'une quelconque des revendications 1 à 4, dans laquelle le support d'indicateur de processus (342) comprend un poteau de montage de pince configuré pour monter une pince (344) maintenant un indicateur de processus (346).

6. Chambre d'isolation d'indicateur de processus selon l'une quelconque des revendications 1 à 4, dans laquelle le support d'indicateur de processus (342) comprend une pince (344) configurée pour maintenir un indicateur de processus (346).

7. Chambre d'isolation d'indicateur de processus selon l'une quelconque des revendications 1 à 4, dans laquelle le support d'indicateur de processus (348) comprend une pince à ressort (348).

8. Chambre d'isolation d'indicateur de processus selon l'une quelconque des revendications 1 à 7, dans laquelle le support d'indicateur de processus est un premier support d'indicateur de processus (342), et le module amovible (304) comprend un second support d'indicateur de processus (348) configuré pour maintenir une bande indicatrice de test supplémentaire.

9. Chambre d'isolation d'indicateur de processus selon l'une quelconque des revendications 1 à 8, dans laquelle le cadre (330) comprend :
un corps principal (332) présentant des surfaces principales opposées (334, 336) ; et
des rails latéraux (338,340) s'étendant perpendiculairement au corps principal (332), les rails latéraux (338, 340) et le corps principal (332) formant un canal, dans laquelle le support d'indicateur de processus (342) est agencé pour fournir l'indicateur de processus au moins partiellement dans le canal.

10. Chambre d'isolation d'indicateur de processus selon la revendication 9, comprenant une ou plusieurs régions découpées (352, 354, 356) s'étendant à travers le corps principal (332), l'une ou les plusieurs régions découpées (352, 354, 356) étant configurées pour permettre l'écoulement de fluide à travers le corps principal (332).

11. Chambre d'isolation d'indicateur de processus selon l'une quelconque des revendications 1 à 10, dans laquelle le capuchon de protection (358) comprend des goussets décalés (372) s'étendant à partir de la surface intérieure (362) de la partie supérieure (360) pour maintenir un espace entre la seconde extrémité (310) du boîtier (302) et le capuchon de protection (358).

12. Processeur de dispositif médical (100), comprenant :
un ensemble bassin (200) comprenant une cuve de bassin (201) ; et
la chambre d'isolation d'indicateur de processus (300) selon l'une quelconque des revendications 1 à 11 disposée dans un trou traversant de la cuve de bassin (201), l'entrée du boîtier de la chambre d'isolation d'indicateur de processus (200) étant positionnée à l'extérieur de la cuve de bassin (201), l'ouverture à la seconde extrémité du boîtier étant positionnée à l'intérieur de la cuve de bassin,
dans lequel la chambre d'isolation d'indicateur de processus (300) est raccordée de manière fluidique à la cuve de bassin (201) de sorte que le fluide puisse s'écouler à travers la chambre d'isolation (300) jusque dans la cuve de bassin (201).

13. Processeur de dispositif médical selon la revendication 12, dans lequel :
l'ensemble de bassin (200) comprend en outre :
un couvercle (214) configuré pour recouvrir sélectivement la cuve de bassin (201) ;
un ou plusieurs raccords (220) en communication fluidique avec le volume interne de la cuve de bassin (201) et configurés pour être raccordés à un dispositif médical traité dans le processeur de dispositif médical (100) ;
une ou plusieurs rampes de pulvérisation (216) en communication fluidique avec le volume intérieur de la cuve de bassin (201) ; et
un drain (224) en communication fluidique avec le volume intérieur de la cuve de bassin (201),
dans lequel le passage de sortie tortueux (374) de la chambre d'isolation d'indicateur de processus (300) est en communication fluidique avec le volume intérieur de la cuve de bassin (201).

14. Processeur de dispositif médical selon la revendication 13, comprenant en outre :
un collecteur (400) ; et
une ligne d'alimentation (124) raccordant le collecteur (400) à l'un ou les plusieurs raccords (220) ;
une pompe (144) et/ou vanne (164) pour réguler le débit de fluide vers l'un ou les plusieurs raccords (220) ;
une ligne d'alimentation (126) raccordant le collecteur (400) à l'une ou les plusieurs rampes de pulvérisation (216) ;
une pompe (146) et/ou vanne (166) pour réguler le débit de fluide vers l'une ou les plusieurs rampes de pulvérisation (216) ;
une ligne d'alimentation (120) raccordant le collecteur (400) à la chambre d'isolation d'indicateur de processus (300) ;
une pompe (140) et/ou vanne (162) pour réguler le débit de fluide vers la chambre d'isolation d'indicateur de processus (300) ;
une ligne de retour (128) raccordant le drain (224) au collecteur (300) ; et
une pompe (148) et/ou vanne (168) pour réguler le débit de fluide du drain (224) de l'ensemble bassin (200) vers le collecteur (300).

15. Processeur de dispositif médical selon la revendication 14, comprenant en outre un contrôleur (600) configuré pour contrôler les vannes et/ou pompes respectives des lignes d'alimentation et la pompe et/ou vanne de la ligne de retour.

16. Processeur de dispositif médical selon la revendication 15, dans lequel le contrôleur (600) est configuré pour exécuter un programme de cycle de traitement (620) stocké dans une mémoire (608), le programme de cycle de traitement (620) comprenant :
un cycle de nettoyage dans lequel une solution comprenant du détergent est mise en circulation à travers la cuve de bassin (201) et le collecteur (400) pendant une durée prédéterminée, en introduisant la solution comprenant du détergent à partir du collecteur (400) vers la cuve de bassin (201) par l'intermédiaire de l'une ou des plusieurs rampes de pulvérisation (216) et de l'un ou des plusieurs raccords (220), et en drainant la solution de la cuve de bassin (201) de retour vers le collecteur (400), la pompe (140) et/ou la vanne (160) associée(s) à la chambre d'isolation d'indicateur de processus (300) étant contrôlée(s) par le contrôleur (600) de sorte que la solution comprenant le détergent ne soit pas introduite à l'intérieur de la chambre d'isolation d'indicateur de processus (300) ; et
un cycle de décontamination dans lequel une solution comprenant un décontaminant est mise en circulation à travers la cuve de bassin (201) et le collecteur (400) pendant une durée prédéterminée, en introduisant la solution comprenant le décontaminant à partir du collecteur (400) vers la cuve de bassin (201) par l'intermédiaire de l'une ou des plusieurs rampes de pulvérisation (216) et de l'un ou des plusieurs raccords (220), et en drainant la solution comprenant le décontaminant de la cuve de bassin (201) de retour vers le collecteur (400), la pompe (140) et/ou la vanne (160) associée(s) à la chambre d'isolation d'indicateur de processus (300) étant contrôlée(s) par le contrôleur de sorte que la solution comprenant le décontaminant soit introduite à l'intérieur de la chambre d'isolation d'indicateur de processus (300).

17. Processus de dispositif médical selon la revendication 16, dans lequel le décontaminant est un désinfectant de haut niveau.

18. Processus de dispositif médical selon la revendication 16, dans lequel le décontaminant est un stérilisant.

19. Processeur de dispositif médical selon la revendication 16, dans lequel le décontaminant est l'acide peracétique ou le glutaraldéhyde.

20. Procédé de détermination de l'efficacité d'un processus de désinfection comprenant les étapes consistant à :
effectuer un cycle de nettoyage dans un processeur de dispositif médical, le processeur de dispositif médical (100) comprenant :
un ensemble bassin (200) comprenant une cuve de bassin (201) ;
un couvercle (214) configuré pour recouvrir sélectivement la cuve de bassin (201) ;
une ou plusieurs buses (218) en communication fluidique avec le volume interne de la cuve de bassin (201) ;
un ou plusieurs raccords (220) en communication fluidique avec le volume interne de la cuve de bassin (201) et configurés pour être raccordés à un dispositif médical traité dans le processeur de dispositif médical (100) ;
une ou plusieurs rampes de pulvérisation (216) en communication fluidique avec le volume intérieur de la cuve de bassin (201) ; et
la chambre d'isolation d'indicateur de processus (300) selon l'une quelconque des revendications 1 à 11 disposée dans un trou traversant de la cuve de bassin (201), l'entrée (314) du boîtier de la chambre d'isolation d'indicateur de processus (302) étant positionnée à l'extérieur de la cuve de bassin (201), l'ouverture (316) à la seconde extrémité (310) du boîtier (302) étant positionnée à l'intérieur de la cuve de bassin (201), dans lequel la chambre d'isolation d'indicateur de processus (300) est raccordée de manière fluidique à la cuve de bassin (201) de sorte que le fluide provenant de la cuve de bassin (201) puisse s'écouler sélectivement à travers la chambre d'isolation (300), dans lequel le cycle de nettoyage comprend l'introduction d'une solution comprenant du détergent à l'intérieur de la cuve de bassin (201) par l'intermédiaire de l'une ou des plusieurs rampes de pulvérisation (216), de l'une ou des plusieurs buses (218) et de l'un ou des plusieurs raccords (220), la solution comprenant le détergent n'étant pas introduite à l'intérieur de la chambre d'isolation d'indicateur de processus (300) pendant le cycle de nettoyage ; et
effectuer un cycle de décontamination comprenant l'introduction d'une solution comprenant un décontaminant à l'intérieur de la cuve de bassin (201) par l'intermédiaire de l'une ou des plusieurs rampes de pulvérisation (216), de l'une ou des plusieurs buses (218) et de l'un ou des plusieurs raccords (220), la solution comprenant le décontaminant étant introduite à l'intérieur de la chambre d'isolation d'indicateur de processus (300) pendant le cycle de décontamination.
